(19) Europäisches Patentamt
European Patent Office
Office européen des brevets

(11) EP 0 896 519 B1

(12) **EUROPÄISCHE PATENTSCHRIFT**

(45) Veröffentlichungstag und Bekanntmachung des Hinweises auf die Patenterteilung:
**09.04.2003 Patentblatt 2003/15**

(21) Anmeldenummer: **97910382.7**

(22) Anmeldetag: **29.09.1997**

(51) Int Cl.[7]: **A61K 7/13**

(86) Internationale Anmeldenummer:
**PCT/EP97/05337**

(87) Internationale Veröffentlichungsnummer:
**WO 98/019660 (14.05.1998 Gazette 1998/19)**

# (54) FÄRBEMITTEL ZUR FÄRBUNG VON KERATINISCHEN FASERN

COLORANTS FOR COLOURING KERATIN FIBRES

COLORANT S'UTILISANT DANS LA COLORATION DE FIBRES KERATINIQUES

(84) Benannte Vertragsstaaten:
**DE ES FR GB IT**

(30) Priorität: **05.11.1996 DE 19645539**

(43) Veröffentlichungstag der Anmeldung:
**17.02.1999 Patentblatt 1999/07**

(73) Patentinhaber: **Wella Aktiengesellschaft**
**64274 Darmstadt (DE)**

(72) Erfinder: **BRAUN, Hans-Jürgen**
**CH-3182 Ueberstorf (CH)**

(56) Entgegenhaltungen:
**EP-A- 0 706 787**
**DE-A- 3 834 142**
**DE-A- 3 942 294**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 896 519 B1

Printed by Jouve, 75001 PARIS (FR)

## Beschreibung

Die vorliegende Erfindung beschreibt eine Zusammensetzung zur Färbung von keratinischen Fasern, insbesonders von menschlichen Haaren, mit einem Gehalt an 2-(2',5'-Diaminophenyl)-ethanol, 2-Methylresorcin, 3-Aminophenol und einem 2-Amino-6-chlor-4-nitrophenol-Derivat.

Auf dem Gebiet der Haarfärbung haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines Oxidationsmittels. Von besonderer Bedeutung sind Haarfärbemittel zur Färbung im Naturtonbereich.

An Oxidationsfarbstoffe, die zur Färbung menschlicher Haare verwendet werden sollen, sind zahlreiche besondere Anforderungen gestellt. Die Farbstoffe müssen in toxikologischer und dermatologischer Hinsicht unbedenklich sowie nicht sensibilisierend sein und Färbungen in der gewünschten Intensität ermöglichen.

Ferner wird für die erzielten Haarfärbungen eine gute Licht-, Dauerwell-, Säure- und Reibeechtheit gefordert. Die Haarfärbungen sollen auch ohne Einwirkung von Licht, Reibung und chemischen Mitteln über einen Zeitraum von mindestens vier bis sechs Wochen stabil bleiben.

Außerdem ist es erforderlich, daß durch Kombination geeigneter Entwickler- und Kupplerkomponenten eine breite Palette verschiedener Farbnuancen erzeugt werden kann.

Ein weiteres Problem besteht in der Praxis bezüglich des unterschiedlichen Aufziehverhaltens der Farbstoffe in Abhängigkeit von der Haarbeschaffenheit, wodurch eine ungleichmäßige Anfärbung der Haare erfolgt. Normalerweise neigen die verwendeten Farbstoffe dazu, auf geschädigte Haarpartien stärker aufzuziehen als auf ungeschädigte Haarpartien. Aus diesem Grunde werden in der Regel die durch übliche Alterungsprozesse und Umwelteinflüsse (zum Beispiel Sonnenlicht, Haarwäsche, Färbe- und Dauerwellbehandlungen) stärker geschädigten Haarspitzen intensiver gefärbt als die weniger geschädigten Haarlängen und der Haaransatz, wodurch ein unnatürliches, ungleichmäßiges und völlig unbefriedigendes Färbeergebnis erhalten wird. Dieses Verhalten macht sich besonders bei hellen Nuancen sehr störend bemerkbar.

Es bestand daher die Aufgabe, ein Oxidationshaarfärbemittel zur Verfügung zu stellen, das eine gleichmäßige und farbsatte Färbung der Haare von den Haarspitzen bis zum Haaransatz im Naturbereich ermöglicht, und gleichzeitig wenig oder gar nicht mutagen und sensibilisierend sowie physiologisch gut verträglich ist.

In der EP-PS 0 686 389 wird die Kombination von 2-(2',5'-Diaminophenyl)ethanol mit 2-Methylresorcin und 3-Aminophenol zur Färbung von Haaren in natürlichen Farbtönen empfohlen. Diese Kombination ergibt jedoch auf ungleichmäßig geschädigten Haaren ein unbefriedigendes Farbresultat. Außerdem sind helle Farbtöne damit nicht nuancierbar.

Weiterhin beschreibt die EP-OS 0 706 787 Oxidationshaarfärbemittel, welche 2-Chlor-6-ethylamino-4-nitrophenol in Kombination mit 1,4-Diaminobenzol und/oder 2,5-Diaminotoluol sowie 2-(2',5'-Diaminophenyl)ethanol und 5-amino-2-methyl-phenol enthalten, wobei weitere Oxidationsfarbstoffvorstufen zugesetzt werden können.

Überraschend wurde nun gefunden, daß der mit der Kombination von 2-(2',5'-Diaminophenyl)ethanol mit 2-Methylresorcin und 3-Aminophenol mit einem 2-Amino-6-chlor-4-nitrophenol gemäß allgemeiner Formel (I) die beschriebenen Probleme mit dem Farbausgleich zwischen Haaransatz und Haarspitze erfolgreich gelöst werden können.

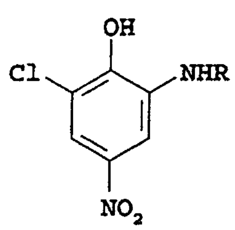

I

In der allgemeinen Formel (I) bedeutet R Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen.

Bevorzugte Derivate gemäß allgemeiner Formel (I) sind
2-Amino-6-chlor-4-nitrophenol, 2-Chlor-6-(ethylamino)-4-nitrophenol und
2-Chlor-6-((2'-hydroxyethyl)amino)-4-nitrophenol.

Die beschriebene Farbstoffkombination ermöglicht eine vom Haaransatz bis zur Haarspitze gleichmäßige Färbung im Naturbereich bis zu leicht modischen Färbungen mit rötlichen Reflexen, ganz besonders auch bei helleren Nuancen. Die vorzüglichen Eigenschaften der neuen Farbstoffkombination zeigen sich besonders auf durch Licht und Wetter geschädigtem oder auf dauergewellten Haaren.

Zur Abrundung des Farbergebnisses sowie zur Erzeugung von speziellen Farbeffekten können dem Haarfärbemittel weitere Oxidationsfarbvorstufen wie Derivate des p-Phenylendiamins sowie direktziehende Farbstoffe, wie zum Beispiel Azofarbstoffe, Anthrachinone oder Nitrobenzolderivate zugesetzt werden.

Die vorstehend beschriebene erfindungsgemäße Kombination von oxidativen Haarfarbvorstufen und gegebenenfalls direktziehenden Farbstoffen werden zur Färbung ein einer geeigneten Farbträgermasse appliziert.

Der Gegenstand der vorliegenden Anmeldung betrifft daher auch ein Mittel zur oxidativen Färbung von Haaren, welches durch Vermischen einer Farbträgermasse mit einem Oxidationsmittel unmittelbar vor der Anwendung hergestellt wird.

Die erfindungsgemäße Farbträgermasse enthält die vorstehend beschriebene erfindungsgemäße Kombination von 2-(2',5'-Diaminophenyl)ethanol mit 2-Methylresorcin, 3-Amino-phenol und einem 2-Amino-6-chlor-4-nitrophenolderivat gemäß allgemeiner Formel (I), als solche oder in Form von physiologisch verträglichen Salzen, beispielsweise als Hydrochloride, Sulfate oder Tartrate oder im Fall von Phenolen als Alkaliphenolate.

Die Gesamtkonzentration an Farbvorstufen in der Farbträgermasse beträgt vorzugsweise 0,1 und 10 Gewichtsprozent, insbesondere 0,2 und 6 Gewichtsprozent.

Das 2-(2',5'-Diaminophenyl)ethanol oder seine Salze sind in einer Konzentration von 0,01 bis 10 Gewichtsprozent, vorzugsweise von 0,05 bis 5 Gewichtsprozent, bezogen auf das Gesamtgewicht der Farbträgermasse, enthalten, während das 2-Methylresorcin, das 3-Aminophenol und das 2-Amino-6-chlor-4-nitrophenol der allgemeinen Formel (I) jeweils in einer Konzentration von 0,01 bis 5 Gewichtsprozent, vorzugsweise von 0,05 bis 3 Gewichtsprozent, bezogen auf das Gesamtgewicht der Farbträgermasse, enthalten ist.

Darüberhinaus können in der Farbträgermasse noch übliche kosmetische Zusätze, beispielsweise Antioxidantien wie Ascorbinsäure, Thioglykolsäure oder Natriumsulfit; Parfümöle; Komplexbildner; Netzmittel und Emulgatoren; Verdicker; Pflegestoffe und andere vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettalkohole, oxethylierte Nonylphenole, Fettsäurealkanolamide, oxethylierte Fettsäureester, ferner Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (bezogen auf die Farbträgermasse), die Verdicker in einer Menge von etwa 0,1 bis 25 Gewichtsprozent (bezogen auf die Farbträgermasse) und die Pflegestoffe in einer Konzentration von etwa 0,1 bis 5,0 Gewichtsprozent (bezogen auf die Farbträgermasse).

Das gebrauchsfertige erfindungsgemäße Haarfärbemittel wird durch Mischen der Farbträgermasse mit einem flüssigen Oxidationsmittel unmittelbar vor der Anwendung hergestellt.

Die Farbträgermasse und das Oxidationsmittel werden hierbei im Gewichtsverhältnis von 5:1 bis 1:3 miteinander vermischt, wobei ein Gewichtsverhältnis von 1:1 bis 1:2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem in der Regel sauer eingestellten Oxidationsmittel auf einen Wert ein, der durch die Alkalimengen in der Farbträgermasse und die Säuremengen im Oxidationsmittel sowie durch das Mischungsverhältnis bestimmt wird. Der pH-Wert des fertigen Haarfärbemittels kann 3 bis 11, vorzugsweise 5 bis 9 betragen.

Für die Einstellung des jeweiligen pH-Wertes der Farbträgermasse und des Oxidationsmittels können je nach dem gewünschten pH-Wert organische und anorganische Säuren, wie zum Beispiel Phosphorsäure, Ascorbinsäure und Milchsäure, oder Alkalien wie zum Beispiel Monoethanolamin, Triethanolamin, 2-Amino-2-methyl-1-propanol, Ammoniak, Natronlauge, Kalilauge oder Tris(hydroxymethyl)-amino-methan, verwendet werden.

Für die Anwendung zur oxidativen Färbung von Haaren vermischt man die vorstehend beschriebene Farbträgermasse unmittelbar vor dem Gebrauch mit einem Oxidationsmittel und trägt eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar auf.

Als Oxidationsmittel kommen hauptsächlich Wasserstoffperoxid oder dessen Additionsverbindungen an Harnstoff, Melamin oder Natriumbromat in Form einer 1- bis 12prozentigen, vorzugsweise 6prozentigen, wäßrigen Lösung in Betracht, wobei Wasserstoffperoxid besonders bevorzugt wird.

Man läßt das erfindungsgemäße Haarfärbemittel bei 15 bis 50 Grad Celsius etwa 10 bis 45 Minuten, vorzugsweise 30 Minuten, lang auf das Haar einwirken, spült sodann das Haar mit Wasser und trocknet es. Gegebenenfalls wird das Haar im Anschluß an diese Spülung mit einem Shampoo gewaschen und/oder mit einer schwachen organischen Säure, wie zum Beispiel Zitronensäure oder Weinsäure, nachgespült. Anschließend wird das Haar getrocknet.
Die nachfolgenden Beispiele sollen den Gegenstand näher erläutern, ohne ihn auf diese Beispiele zu beschränken.

**Beispiele**

**Beispiel 1:** Haarfärbelösung

| | |
|---|---|
| 2,6 g | 2-(2',5'-Diaminophenyl)-ethanol-sulfat |
| 0,6 g | 2-Methylresorcin |
| 0,5 g | 3-Aminophenol |
| 0,2 g | 2-Amino-6-chlor-4-nitrophenol |
| 10,0 g | Isopropanol |
| 10,0 g | Laurylalkohol-diglykolether-sulfat-Natriumsalz, 28prozentige wäßrige Lösung |
| 10,0 g | Ammoniak, 25prozentige wäßrige Lösung |
| 0,3 g | Ascorbinsäure |
| 65,8 g | Wasser, vollentsalzt |
| 100,0 g | |

Zur Anwendung werden 10 g Haarfärbelösung mit 10 g Wasserstoffperoxid-Lösung (6prozentige wäßrige Lösung) gemischt. Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.
Das Haar hat einen gleichmäßig walnußbraunen Farbton angenommen.

**Beispiel 2:** Haarfärbecreme

| | |
|---|---|
| 0,3 g | 2-(2',5'-Diaminophenyl)-ethanol-sulfat |
| 0,1 g | 2-Methylresorcin |
| 0,1 g | 3-Aminophenol |
| 0,2 g | 2-Chlor-6-(ethylamino)-4-nitrophenol |
| 15,0 g | Cetylalkohol |
| 3,5 g | Laurylalkohol-diglykolether-sulfat-Natriumsalz, 28prozentige wäßrige Lösung |
| 3,0 g | Ammoniak, 25prozentige wäßrige Lösung |
| 0,3 g | Natrimsulfit, wasserfrei |
| 77,5 g | ,Wasser, vollentsalzt |
| 100,0 g | |

Zur Anwendung werden 10 g Haarfärbecreme mit 10 g Wasserstoffperoxid-Lösung (6prozentige wäßrige Lösung) gemischt. Das erhaltene Oxidationshaarfärbemittel wird auf durch Wind und Wetter geschädigte Haare aufgetragen. Nach einer Einwirkungszeit von 30 Minuten bei 40 °C werden die Haare mit Wasser gespült, schamponiert und getrocknet.
Das Haar ist gleichmäßig vom Ansatz bis zur Spitze in einem Kupfer-Ton gefärbt.
Die angegeben Prozentwerte stellen soweit nicht anders angegeben Gewichtsprozente dar.

**Patentansprüche**

1. Haarfarbträgermasse **dadurch gekennzeichnet, daß** sie eine Kombination aus 2-(2',5'-Diaminophenyl)ethanol, 2-Methylresorcin, 3-Aminophenol und einem 2-Amino-6-chlor-4-nitrophenol der allgemeinen Formel (I) enthält,

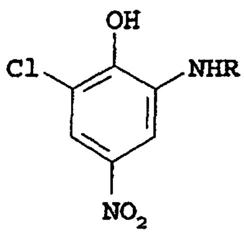

I

,

worin R Wasserstoff, eine geradkettige oder verzweigte Alkylgruppe mit 1 bis 6 Kohlenstoffatomen, eine geradkettige oder verzweigte Hydroxyalkylgruppe mit 2 bis 4 Kohlenstoffatomen oder eine geradkettige oder verzweigte Polyhydroxyalkylgruppe mit 3 bis 4 Kohlenstoffatomen bedeutet.

2. Haarfarbträgermasse nach Anspruch 1, **dadurch gekennzeichnet, daß** das 2-Amino-6-chlor-4-nitrophenol der allgemeinen Formel (I) ausgewählt ist aus 2-Amino-6-chlor-4-nitrophenol, 2-Chlor-6-(ethylamino)-4-nitrophenol und 2-Chlor-6-((2'-hydroxyethyl)amino)-4-nitrophenol.

3. Haarfarbträgermasse nach Anspruch 1 oder 2, **dadurch gekennzeichnet, daß** das 2-(2',5'-Diaminophenyl)-ethanol in Form seines Additionssalzes mit einer anorganischen oder organischen Säure enthalten ist.

4. Haarfarbträgermasse nach einem der Ansprüche 1 bis 3, **dadurch gekennzeichnet, daß** das 2-(2',5'-Diaminophenyl)ethanol oder sein Salz in einer Konzentration von 0,01 bis 10 Gewichtsprozent, bezogen auf das Gesamtgewicht der Zusammensetzung, enthalten ist.

5. Haarfarbträgermasse nach einem der Ansprüche 1 bis 4, **dadurch gekennzeichnet, daß** das 2-Methylresorcin, das 3-Aminophenol und das 2-Amino-6-chlor-4-nitrophenol der allgemeinen Formel (I) jeweils in einer Konzentration von 0,01 bis 5 Gewichtsprozent, bezogen auf das Gesamtgewicht der Haarfarbträgermasse, enthalten ist.

6. Haarfarbträgermasse nach einem der Ansprüche 1 bis 5, **dadurch gekennzeichnet, daß** die Farbvorstufen in einer Gesamtkonzentration von 0,01 bis 10 Gewichtsprozent, bezogen auf das Gesamtgewicht der Zusammensetzung, enthalten sind.

7. Haarfarbträgermasse nach einem der Ansprüche 1 bis 6, **dadurch gekennzeichnet, daß** sie übliche kosmetische Zusätze enthält.

8. Haarfärbemittel, **dadurch gekennzeichnet, daß** es durch Vermischen der Haarfarbträgermasse nach einem der Ansprüche 1 bis 7 mit einem Oxidationsmittel unmittelbar vor der Anwendung hergestellt wird.

9. Haarfärbemittel nach Anspruch 8, **dadurch gekennzeichnet, daß** die Haarfarbträgermasse mit dem Oxidationsmittel in einem Verhältnis von 5:1 bis 1:3 vermischt wird.

10. Haarfärbemittel nach Anspruch 8 oder 9, **dadurch gekennzeichnet, daß** es einen pH-Wert von 3 bis 11 aufweist.

11. Verfahren zum oxidativen Färben von Haaren, **dadurch gekennzeichnet, daß** ein Haarfärbemittel nach einem der Ansprüche 8 bis 10 auf das Haar aufgetragen wird, bei einer Temperatur von 15 bis 50 °C etwa 10 bis 45 Minuten lang einwirken gelassen wird, das Haar anschließend mit Wasser gespült wird, gegebenenfalls schamponiert wird und sodann getrocknet wird.

## Claims

1. Hair colour carrier mass **characterized in that** it comprises a combination of 2-(2',5'-diaminophenyl)ethanol, 2-methylresorcinol, 3-aminophenol and a 2-amino-6-chloro-4-nitrophenol of the general formula (I)

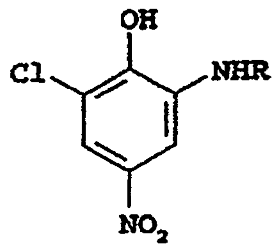

I

in which R is hydrogen, a straight-chain or branched alkyl group having 1 to 6 carbon atoms, a straight-chain or branched hydroxyalkyl group having 2 to 4 carbon atoms or a straight-chain or branched polyhydroxyalkyl group having 3 to 4 carbon atoms.

2. Hair colour carrier mass according to Claim 1, **characterized in that** the 2-amino-6-chloro-4-nitrophenol of the general formula (I) is chosen from 2-amino-6-chloro-4-nitrophenol, 2-chloro-6-(ethylamino)-4-nitrophenol and 2-chloro-6-((2'-hydroxyethyl)amino)-4-nitrophenol.

3. Hair colour carrier mass according to Claim 1 or 2, **characterized in that** the 2-(2',5'-diaminophenyl)ethanol is present in the form of its addition salt with an inorganic or organic acid.

4. Hair colour carrier mass according to one of Claims 1 to 3, **characterized in that** the 2-(2',5'-diaminophenyl)ethanol or its salt is present in a concentration of from 0.01 to 10 percent by weight, based on the total weight of the composition.

5. Hair colour carrier mass according to one of Claims 1 to 4, **characterized in that** the 2-methylresorcinol, the 3-aminophenol and the 2-amino-6-chloro-4-nitrophenol of the general formula (I) is present in each case in a concentration of from 0.01 to 5 percent by weight, based on the total weight of the hair colour carrier mass.

6. Hair colour carrier mass according to one of Claims 1 to 5, **characterized in that** the dye precursors are present in a total concentration of from 0.01 to 10 percent by weight, based on the total weight of the composition.

7. Hair colour carrier mass according to one of Claims 1 to 6, **characterized in that** it comprises customary cosmetic additives.

8. Hair colorant **characterized in that** it is prepared directly prior to application by mixing the hair colour carrier mass according to one of Claims 1 to 7 with an oxidizing agent.

9. Hair colorant according to Claim 8, **characterized in that** the hair colour carrier mass is mixed with the oxidizing agent in a ratio of from 5:1 to 1:3.

10. Hair colorant according to Claim 8 or 9, **characterized in that** it has a pH of from 3 to 11.

11. A method for the oxidative colouring of hair, **characterized in that** a hair colorant according to one of Claims 8 to 10 is applied to the hair, left to act at a temperature of from 15 to 50°C for about 10 to 45 minutes, and the hair is then rinsed with water, optionally shampooed and then dried.

## Revendications

1. Matière colorante pour cheveux, **caractérisée en ce qu'**elle contient une association de 2-(2',5'-diaminophényl) éthanol, 2-méthylrésorcinol, 3-aminophénol et d'un 2-amino-6-chloro-4-nitrophénol de formule générale (I)

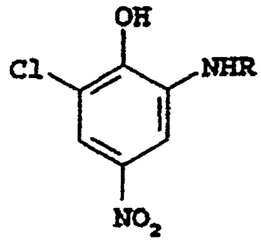

I

,

dans laquelle R représente un atome d'hydrogène, un groupe alkyle à chaîne droite ou ramifiée ayant de 1 à 6 atomes de carbone, un groupe hydroxyalkyle à chaîne droite ou ramifiée ayant de 2 à 4 atomes de carbone ou un groupe polyhydroxyalkyle à chaîne droite ou ramifiée ayant 3 ou 4 atomes de carbone.

2. Matière colorante pour cheveux selon la revendication 1, **caractérisée en ce que** le 2-amino-6-chloro-4-nitrophénol de formule générale (I) est choisi parmi le 2-amino-6-chloro-4-nitrophénol, le 2-chloro-6-(éthylamino)-4-nitrophénol et le 2-chloro-6-((2'-hydroxyéthyl)amino)-4-nitrophénol.

3. Matière colorante pour cheveux selon la revendication 1 ou 2, **caractérisée en ce que** le 2-(2',5'-diaminophényl) éthanol est utilisé sous forme de son sel d'addition avec un acide organique ou minéral.

4. Matière colorante pour cheveux selon l'une quelconque des revendications 1 à 3, **caractérisée en ce que** le 2-(2',5'-diaminophényl)éthanol ou son sel est contenu à une concentration de 0,01 à 10 % en poids, par rapport au poids total de la composition.

5. Matière colorante pour cheveux selon l'une quelconque des revendications 1 à 4, **caractérisée en ce que** le 2-méthylrésorcinol, le 3-aminophénol et le 2-amino-6-chloro-4-nitrophénol de formule générale (I) sont contenus chacun à une concentration de 0,01 à 5 % en poids, par rapport au poids total de la matière colorante pour cheveux.

6. Matière colorante pour cheveux selon l'une quelconque des revendications 1 à 5, **caractérisée en ce que** les précurseurs de colorants sont utilisés à une concentration totale de 0,01 à 10 % en poids, par rapport au poids total de la composition.

7. Matière colorante pour cheveux selon l'une quelconque des revendications 1 à 6, **caractérisée en ce qu'**elle contient des additifs cosmétiques usuels.

8. Produit de teinture pour cheveux, **caractérisé en ce qu'**il est préparé immédiatement avant l'emploi, par mélange d'une matière colorante pour cheveux selon l'une quelconque des revendications 1 à 7, avec un oxydant.

9. Produit de teinture pour cheveux selon la revendication 8, **caractérisé en ce que** la matière colorante pour cheveux est mélangée avec l'oxydant en un rapport de 5:1 à 1:3.

10. Produit de teinture pour cheveux selon la revendication 8 ou 9, **caractérisé en ce qu'**il présente un pH de 3 à 11.

11. Procédé de teinture des cheveux par oxydation, **caractérisé en ce qu'**on applique sur les cheveux un produit de teinture pour cheveux selon l'une quelconque des revendications 8 à 10, on laisse agir pendant environ 10 à 45 minutes à une température de 15 à 50°C, puis on rince les cheveux à l'eau, éventuellement on les shampooine et ensuite on les sèche.